# EUROPEAN PATENT SPECIFICATION

(11) **EP 0 884 308 B1**
(45) Date of publication and mention of the grant of the patent: **16.04.2003**
(21) Application number: 98201381.5
(22) Date of filing: 28.04.1998
(51) Int. Cl.: C07C 401/00, A61K 31/59

(54) **A method of preparing 16-dehydro vitamin D compounds**
Verfahren zur Herstellung von 16-Dehydro Vitamin D Verbindungen
Procédé de préparation de composés de 16-déhydro vitamine D

(30) Priority: 02.05.1997 EP 97201334
(43) Date of publication of application: 16.12.1998
(73) Proprietor: DUPHAR INTERNATIONAL RESEARCH B.V, 1381 CP Weesp (NL)
(72) Inventor: v.d. Velde, J.P., 1380 AC Weesp (NL); Halkes, S.J., 1380 AC Weesp (NL); Zorgdrager, J., 1380 AC Weesp (NL); del los Angeles Rey, M., 1380 AC Weesp (NL); Mourino, A., 1380 AC Weesp (NL); Fall, Y., 1380 AC Weesp (NL); Halkes, K.M., 1380 AC Weesp (NL); Fernandez-Gacio, A., 1380 AC Weesp (NL)
(74) Representative: Muis, Maarten

(56) References cited:
- EP-A- 0 325 279
- EP-A- 0 599 114
- EP-A- 0 646 576
- EP-A- 0 654 467
- EP-A- 0 717 034
- US-A- 5 384 314
- CHEMICAL ABSTRACTS, vol. 124, no. 5, 29 January 1996 Columbus, Ohio, US; abstract no. 45911t, XP002039835 & M.G. BISCHOF ET AL: J. PHARMACOL. EXP. THER., vol. 275, no. 3, 1995, pages 1254-1260,

## Description

The invention relates to a new method of preparing 16-dehydro vitamin D compounds, to a group of new 16-dehydro vitamin D compounds and to their use in pharmacology.

It is generally known, that vitamin D compounds or vitamin D related compounds ("vitamin D compounds") have a strong biological activity and may be used in all those cases in which problems with the calcium metabolism play a part. A few years ago it was found that various active vitamin D compounds also have other pharmacotherapeutic activities and may be used successfully, for example, for the treatment of certain skin and bone diseases, for cosmetic applications and for treating diseases which are related to cell differentiation, cell proliferation or imbalance in the immune system, including diabetes mellitus, hypertension and inflammatory diseases such as rheumatoid arthritis and asthma. In addition, these compounds may be used in various veterinary applications, and for diagnostic purposes.

It is therefore of the utmost importance to have the disposal of an arsenal of active vitamin D compounds for the above various application fields so as to be able to make the best possible choice of a vitamin D compound for the application in view.

According to the method of the present invention 16-dehydro vitamin D compounds of the general formula I 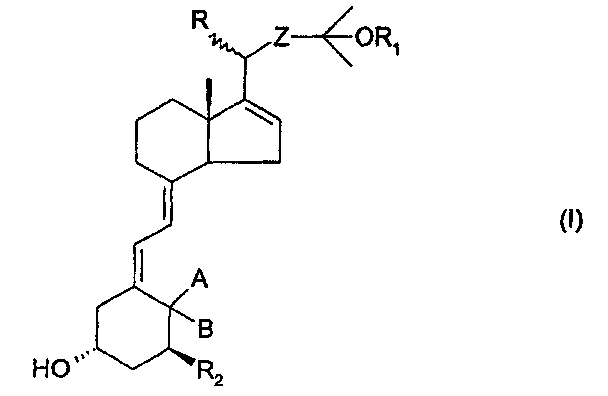 wherein:
- R has the epi-configuration or the normal configuration and represents straight or branched C₁₋₆-alkyl, C₂₋₆-alkenyl, C₃₋₆-cycloalkyl, or an aromatic group optionally substituted with halogen, C₁₋₃-alkyl or alkoxy,
- Z is a saturated or unsaturated straight hydrocarbon group or oxahydrocarbon group containing 3-6 C-atoms which may be substituted with OH, F, alkyl (C₁₋₃) or cycloalkyl (C₃₋₅),
- R₁ is hydrogen or an hydroxy protecting group,
- R₂ is hydrogen or hydroxy, and
- A and B represent hydrogen or methyl, or together form the methylene group,
can easily be obtained in good yield.

The compounds as defined above having formula I wherein the symbols have the meanings given above, with the proviso that when R is methyl and Z is a straight hydrocarbon group containing 3 C-atoms this group R has the epi-configuration, are new compounds.

The new modified vitamin D compounds of the invention, presented by the general formula I, are valuable substances. The biological results indicate that these compounds are promising as biologically active substances and may be used in all above-mentioned pharmacotherapeutic indications, more in particular for the treatment of osteoporosis, renal osteodystrophy, osteomalacia, skin disorders such as psoriasis (and other hyperproliferative skin diseases), eczema and dermatitis, myopathy, leukaemia, breast and colon cancer, osteosarcomas, squamous cell carcinomas, melanoma, certain immunological disorders, and transplant rejections. For this application, the new compounds of the invention may be incorporated, in effective amounts, in pharmaceutical compositions, comprising in addition pharmaceutical acceptable carriers and auxiliary substances. Furthermore, the new vitamin D compounds of the invention may be used for wound healing and may be incorporated in cosmetic compositions, such as creams, lotions, ointments and the like, in order to preserve, condition and/or protect the skin and to improve various skin conditions, such as wrinkles, dry skin, skin slackness and insufficient sebum secretion. The new vitamin D compounds may also be used for diagnostic purposes.

Preferred compounds are vitamin D compounds of the general formula I, wherein Z is the group (CH₂)₃, R₁ is hydrogen, R₂ is hydroxy, and R has the epi-configuration and represents C₁₋₄-alkyl, cyclopropyl or phenyl, and A+B represent methylene.

The invention relates to a new method of preparing 16-dehydro vitamin D compounds of the above formula I by reacting a compound of the general formula II 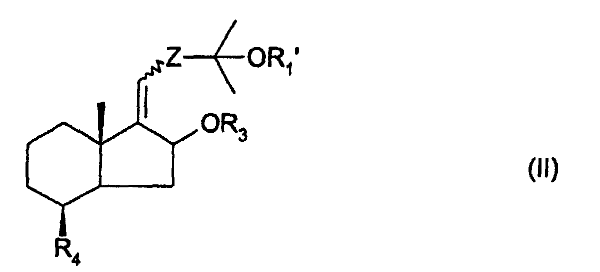 wherein Z has the above meaning, R'₁ is a hydroxy protecting group, R₃ is a carbamate group or a pivalate group, and R₄ is a protected hydroxy group or a group of the formula III 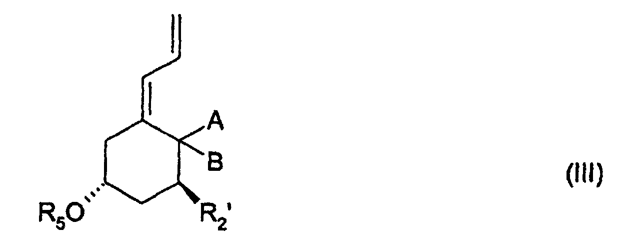 wherein A and B have the above meaning, R₅ is a hydroxy protecting group, and R'₂ is hydrogen or a hydroxy protecting group,
with a suitable cuprate reagent for introducing group R to produce a compound of the formula IV 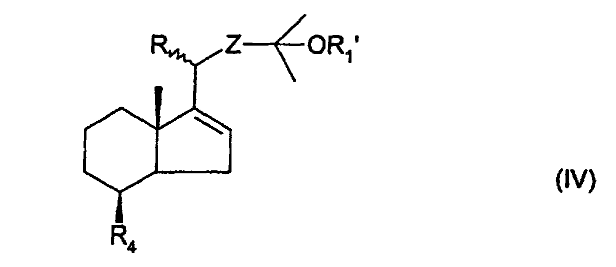 wherein R, R'₁, Z and R4 have the above meaning, followed by converting the obtained compound having formula IV into a compound having formula I in a manner known per se, as illustrated in the Examples.

Hydroxy groups in the above intermediates or reactants may be protected by a reaction with a suitable esterification or etherification agent. A suitable esterification agent is an alkylchlorocarbonate having 2 to 5 carbon atoms, or an aromatic carboxylic acid or saturated aliphatic carboxylic acid having 1 to 4 carbon atoms such as benzoic, or a derivative of such acids suitable for the esterification reaction. In order to protect the hydroxy group in the form of an ether, in principle any etherification agent known for this purpose is suitable: for example, a methoxymethylating agent (such as methoxymethylchloride), a trialkylsilylimidazole, a trialkylsilylhalide, a trialkyl-silyltriflate, a diphenylalkylsilylhalide, or a diphenylalkylsilyltriflate, or a derivative thereof, the alkyl groups of which have 1 to 6 carbon atoms. Particularly suitable for this purpose are trimethylsilylchloride, tert.-butyldimethylsilylchloride, dimethyl-(1,1,2-trimethylpropyl)-silylchloride, tert.-butyldimethylsilyl triflate, or trimethylsilyl-imidazole, because these etherification agents readily react with the hydroxy group(s) to be protected to form an ether function, which on the one hand is sufficiently stable under the conditions of the reaction or reactions in view, but on the other hand can easily be removed [deprotection] to recover the original hydroxy group; tert.-butyldimethylsilylchloride or triflate is to be preferred, because the tert.-butyldimethylsilyl group has been found to be excellently suitable as a protective group.

The starting compounds having formula II can be obtained by converting a corresponding compound wherein the group OR₃ is hydrogen (which compounds are described in EP. 0717034) in a manner known per se into the desired compound II (see Example 3).

The obtained compounds having formula IV wherein R₄ is a protected hydroxy group can be converted into a compound having formula I in a manner known per se by deprotecting group R₄ and oxidising the deprotected hydroxy group to the keto group, and converting the obtained keto-compound
(a) with a Wittig reagent of the general formula V 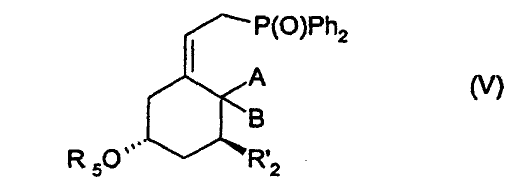 wherein A, B, R₅ and R'₂ have the above meanings; or
(b) after enolization and derivatisation of the enolic hydroxy group, with an enyne compound of the formula VI 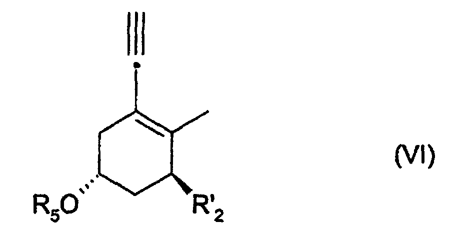 wherein R'₂ and R₅ have the above meaning, followed by hydrogenation and isomerization to produce a compound of formula I wherein A+B form the methylene group;
followed by deprotection of hydroxy group(s).
The enolic hydroxy group in compound VI is preferably derivatized by reaction with N-phenyltriflimide to produce the corresponding triflate.

The conversion of a compound having formula II into a compound of the formula IV is carried out with a so-called cuprate reagent.
The cuprate reagent to be used depends on the meaning of group R in the desired compound having formula I as appears from the following Scheme A (when R₄ is protected hydroxy) and Scheme B (when R₄ is a group of formula III)

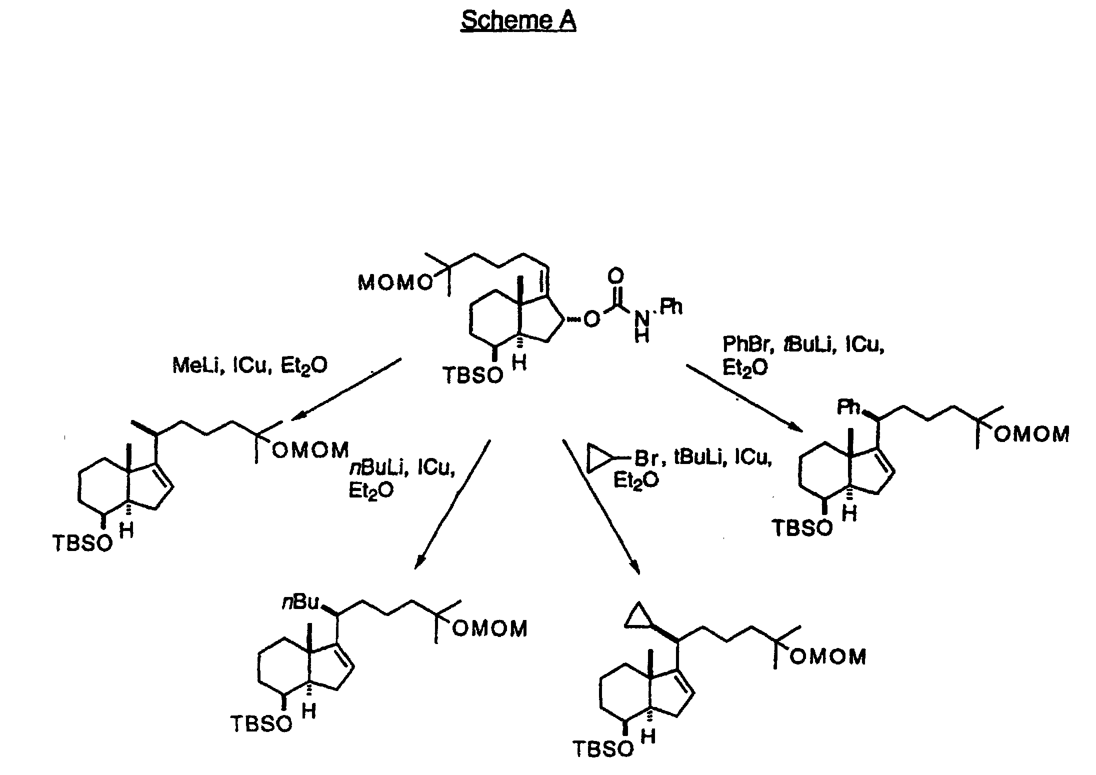

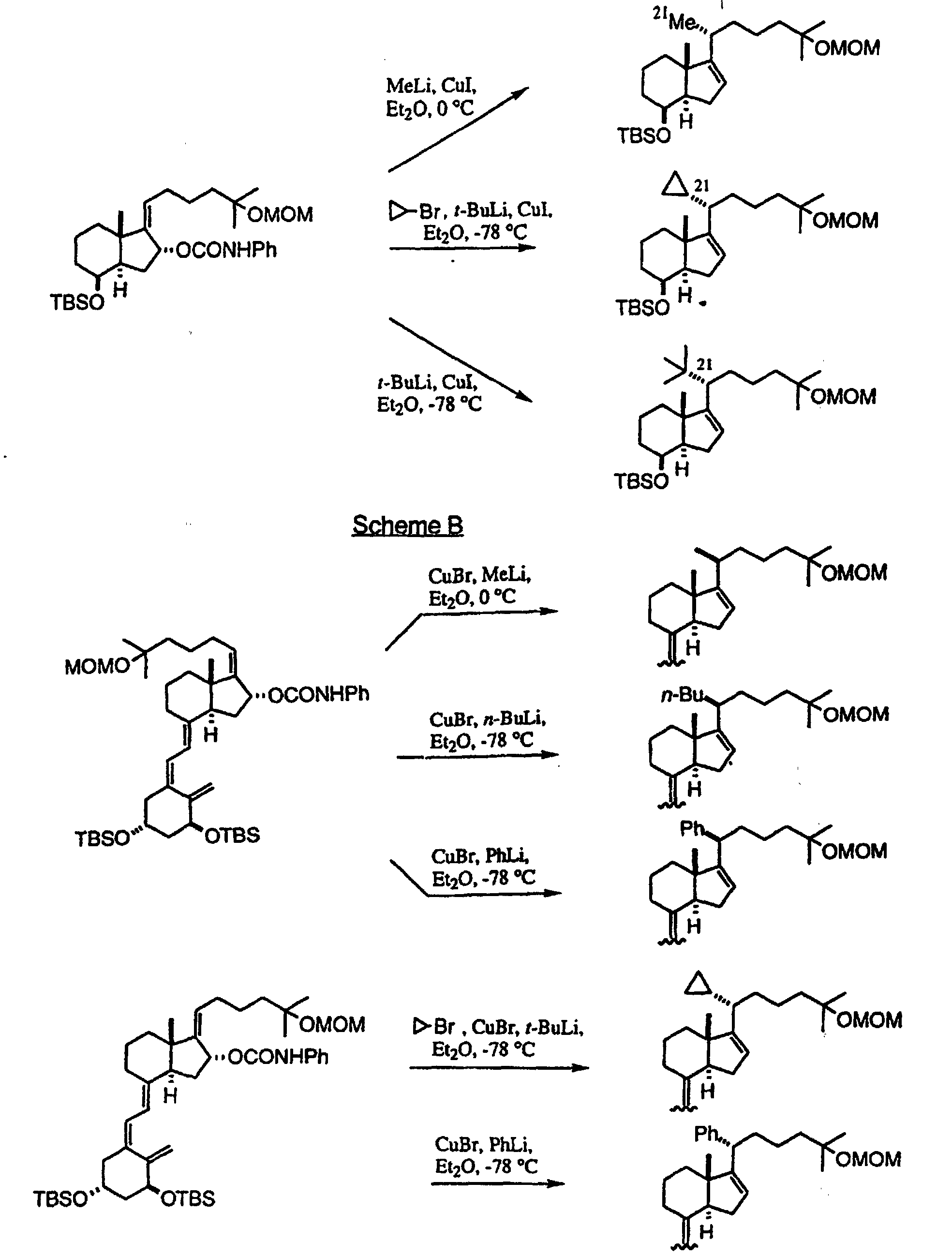

The conversion of a compound of formula IV obtained according to Scheme A, after selective deprotection and oxidation of the hydroxy group to the keto group, with a Wittig reagent is indicated in Scheme C:

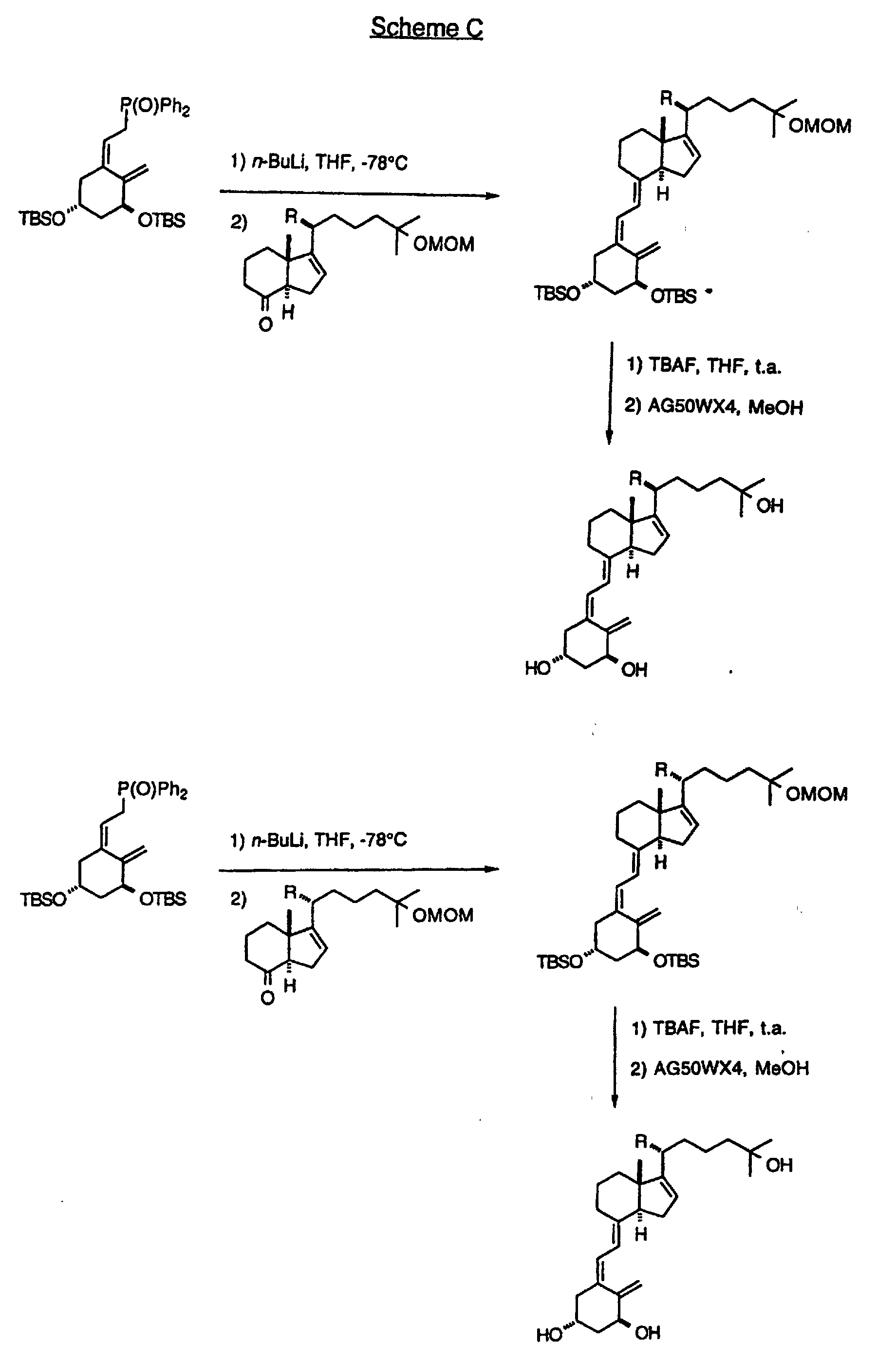

### Example 1

### preparation of compound 2 from compound 1.

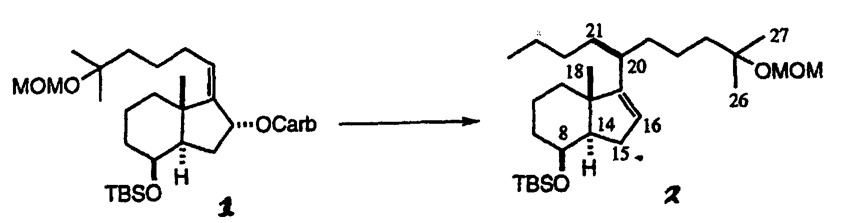

To a cooled (-78 °C) suspension of 379 mg of Cul (1.99 mmol) in 8 ml of dry Et₂O under an argon atmosphere, was added 1.59 ml of a 2.5 M solution of *n*-BuLi in hexanes (3.97 mmol). After stirring for 1 hr, 150 mg of **1** (0.26 mmol) in 4 ml dry Et₂O was added. The reaction mixture was allowed to reach room temperature and stirring was continued for 12 hrs in the dark. The reaction was quenched by the addition of 10 ml of a saturated NH₄Cl solution. After separation of the layers, the aqueous layer was extracted with Et₂O (3 x 10 ml). The combined organic layers were washed with 15 ml of a saturated NaHCO₃ solution, dried, filtered and concentrated at reduced pressure. The residue was purified by flash chromatography (Eluent 1% EtOAC/hexane) resulting in 113.1 mg of **2** as a colourless oil (88%, Rf = 0.76; 20% EtOAc/hexane).

¹H-NMR (CDCl₃,250 MHz, δ): 5.22 (1 H, t, J=1.1 Hz, H-16),4.69 (2 H, s, OCH₂O), 4.06 (1 H, m, H-8),3.38 (3 H, s, MeO), 2.22 (1 H, m, H-14),1.98-1.59 (3 H, m, H-15 and H-20), 1.18 (6 H, s, Me-26 and Me-27), 0.98 (3 H, s, Me-18), 0.85 (11 H, s, t- BuSi and CH₂-21), 0.04 (6 H, s, Me₂Si)

According to a similar procedure the following compounds of the formula IV wherein Z is (CH₂)₃ and R₁'= MOM have been prepared:
a) R= epi-phenyl; R₄=TBSO
   ¹H-NMR (CDCl₃, 250 MHz, δ): 7.28-7.12 (5 H, m, Ar), 5.49 (1 H, t, J=1.5 Hz, H-16), 4.65 (2 H, s, OCH₂O), 4.07 (1 H, m, H-8), 3.29 (3 H, s, MeO), 3.23 (1 H, t, J=7.6 Hz, H-20),2.25 (1 H, m, H-14),1.84 (2 H, m, H-15),1.14 (6 H, s, Me-26 and Me-27), 0.84 (9 H, s, t- BuSi), 0.68 (3 H, s, Me-18), 0.045 (6 H, s, Me₂Si).
b)=epi-cycloppropyl; R₄=TBSO
   ¹H-NMR (CDCl₃,250 MHz, δ): 5.37 (1 H, t, J=1.1 Hz, H-16),4.67 (2 H, s, OCH₂O), 4.07 (1 H, m, H-8),3.33 (3 H, s, MeO), 2.23 (1 H, m, H-14),1.90-1.54 (3 H, m, H-15 and H-20),1.18 (6 H, s, Me-26 and Me-27), 0.99 (3 H, s, Me-18), 0.86 (9 H, s, t- BuSi), 0.80 (1 H, m, H-21), 0.49 (1 H, m, CH₂-cyclopropyl), 0.36 (1 H, m, CH₂-cyclopropyl), 0.10 (1 H, m, CH₂-cyclopropyl), 0.00 (6 H, s, Me₂Si), -0.08 (1 H, m, CH₂- cyclopropyl).
c) R=epi-methyl; R₄=TBSO
   ¹H-NMR (CDCl₃, 250 MHz, δ): 5.29 (1 H, t, J=1.2 Hz, H-16), 4.70 (2 H, s, OCH₂O), 4.09 (1 H, m, H-8),3.36 (3 H, s, MeO), 2.24-2.05 (4 H, m, H-20, H-15 and H-14),1.19 (6 H, s, Me-26 and Me-27), 1.03 (3 H, d, J=6.8 Hz, Me-21), 1.02 (3 H, s, Me-18), 0.85 (9 H, s, t- BuSi), 0.01 (6 H, s, Me₂Si).
d) R=normal-methyl; R₄=TBSO
   ¹H-NMR (CDCl₃,250 MHz, δ): 5.25 (1H, t, J=1.5 Hz, H-16), 4.70 (2 H, s, OCH₂O), 4.08 (1 H, d, J=2.2 Hz, H-8), 3.36 (3 H, s, MeO), 2.22-1.90 (4 H, m, H-14, H-15 and H-20), 1.20 (6 H, s, Me-26 and Me-27), 1.01 (3 H, s, Me-18), 0.97 (3 H, d, J=6.8 Hz, Me-21), 0.89 (9 H, s, t- BuSi), 0.03 (3 H, S, Me₂Si), 0.02 (3 H, s, Me₂Si).
e) R=normal-tert.butyl; R₄=TBSO
   ¹H-NMR (CDCl₃,250 MHz, δ): 5.32 (1H, t, J=1.1 Hz, H-16),4.68 (2 H, s, OCH₂O),-4.09 (1 H, m, H-8),3.37 (3 H, s, MeO), 2.23 (1 H, m, H-14),1.98-1.61 (3 H, m, H-15 and H-20), 1.19 (6 H, s, Me-26 and Me-27), 1.04 (3 H, s, Me-18), 0.88 (9 H, s, t-Bu), 0.86 (9 H, s, t-BuSi), 0.01 (6 H, s, Me₂Si).
f) R=normal-cyclopropyl; R₄=TBSO
   ¹H-NMR (CDCl₃,250 MHz, δ): 5.31 (1H, t, J=1.5 Hz, H-16),4.69 (2 H, s, OCH₂O), 4.08 (1 H, m, H-8), 3.36 (3 H, s, MeO), 2.25 (1 H, m, H-14), 1.90-1.62 (3 H, m, H-15 and H-20), 1.20 (6 H, s, Me-26 and Me-27), 1.06 (3 H, s, Me-18), 0.89 (9 H, s, t- BuSi), 0.88-0.35 (5 H, m, cyclopropyl), 0.026 (6 H, s, Me₂Si).
g) R= epi-methyl; R₄= 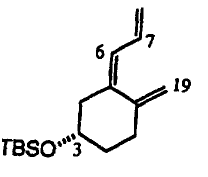
   ¹H-NMR (CD₂Cl₂, 250 MHz, δ): 6.16, 6.09 (2 H, AB, J=11.2 Hz, H-6 and H-7), 5.30 (1 H, s broad, H-16), 5.01 (1 H, d, J=1.2 Hz, H-19_{E}), 4.76 (1 H, d, J=1.6 Hz, H-19_{Z}), 4.63 (2 H, s, OCH₂O),3.84 (1 H, m, H-3),3.29 (3 H, s, MeO), 2.80 (1 H, dd, 1=12.5Hz and 4.4 Hz, H-14), 1.16 (6 H, s, Me-26 and Me-27), 1.02 (3 H, d, J=6.9 Hz, Me-21), 0.87 (9 H, s, t-BuSi), 0.67 (3 H, s, Me-18), 0.05 (3 H, s, MeSi), 0.04 (3 H, s, MeSi).
h) R=epi-n.butyl; R₄= 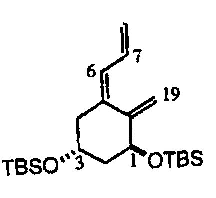 ¹H-NMR (CD₂Cl₂,250 MHz, δ): 6.25,6.10 (2 H, AB, J=11.1 Hz, H-6 and H-7),5.27 (1 H, s broad, H-16),5.19 (1 H, s broad, H-19_{E}), 4.85 (1 H, d, J=2.5 Hz, H-19_{Z}), 4.63(2 H, s, OCH₂O), 4.37 (1 H, m, H-1), 4.18 (1 H, m, H-3), 3.29 (3 H, s, MeO), 2.81(1 H, m, H-14), 1.15 (6 H, s, Me-26 and Me-27), 0.87 (2 H, m, H-21), 0.86 (18 H, s, 2 t-BuSi), 0.62 (3 H, s, Me-18), 0.06 (3 H, s, MeSi), 0.05 (3 H, s, MeSi), -0.10 (3 H, s, MeSi), -0.14 (3 H, s, MeSi).
i) R=normal-cyclopropyl; R₄= 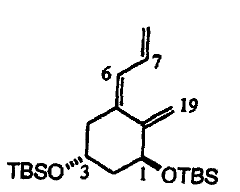
   ¹H-NMR (CDCl₃,250 MHz, δ): 6.26,6.12 (2 H, AB, J=11.7 Hz, H-6 and H-7),5.35 (1 H, m, H-16), 5.19 (1 H, s broad, H-19_{E}), 4.86 (1 H, s broad, H-19_{Z}), 4.63 (2 H, s, OCH₂O), 4.39 (1 H, m, H-1), 4.19 (1 H, m, H-3), 3.28 (3 H, s, MeO), 2.80 (1 H, m, H-14),1.14 (6 H, s, Me-26 and Me-27), 0.88 (18 H, s, 2 t-BuSi), 0.80 (1 H, m, H-21), 0.72 (3 H, s, Me-18), 0.45 (2 H, m, CH₂-cyclopropyl), 0.09 (2 H, m, CH₂-cyclopropyl), 0.06 (6 H, s, Me₂Si), 0.057 (6 H, s, Me₂Si).
j) R=normal-phenyl; R₄=

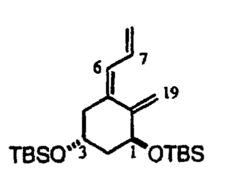

¹H-NMR (CDCl₃, 250 MHz, δ): 7.28-7.14 (5 H, m, Ar), 6.23, 6.12 (2 H, AB, J=11.1 Hz, H-6 and H-7),5.56 (1 H, m, H-16),5.20 (1 H, s broad, H-19_{E}), 4.87 (1 H, s broad, H-19_{Z}), 4.60 (2 H, s, OCH₂O), 4.39 (1 H, m, H-1), 4.19 (1 H, m, H-3), 3.24 (3 H, s, MeO), 2.75 (1 H, m, H-14), 1.12 (6 H, s, Me-26 and Me-27), 0.89 (9 H, s, t-BuSi), 0.87 (9 H, s, t-BuSi), 0.70 (3 H, s, Me-18), 0.08 (6 H, s, Me₂Si), 0.07 (6 H, s, Me₂Si).

### Example 2

### (i) preparation of compound 4 from compound 3

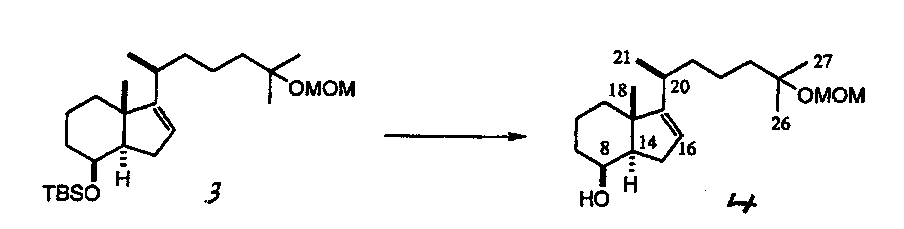

To a solution of 70 mg of **3** (0.16 mmol) in 0.5 ml dry THF under an argon atmosphere, was added 0.79 ml of a 1M solution of TBAF in THF (0.79 mmol). The mixture was heated to reflux and stirring was continued for 12 hrs. The reaction was quenched by the addition of 4 ml of a saturated NaHCO₃ solution. After separation of the layers, the aqueous layer was extracted with Et₂O (3 x 10 ml). The combined organic layers were dried, filtered and concentrated at reduced pressure. The residue was purified by flash chromatography (Eluent 10% EtOAC/hexane) resulting in 50.6 mg of **4** as a colourless oil (98%, Rf = 0.53; 20% EtOAc/hexane).

¹H-NMR (CDCl₃, 250 MHz, δ): 5.30 (1 H, t, J=1.2 Hz, H-16),4.66 (2 H, s, OCH₂O), 4.09 (1 H, m, H-8),3.33 (3 H, s, MeO), 2.28-2.05 (4 H, m, H-20, H-15 and H-14),1.17 (6 H, s, Me-26 and Me-27),1.03 (3 H, d, J=6.8 Hz, Me-21),1.02 (3 H, s, Me-18).

### (ii) preparation of compound 5 fom compound 4

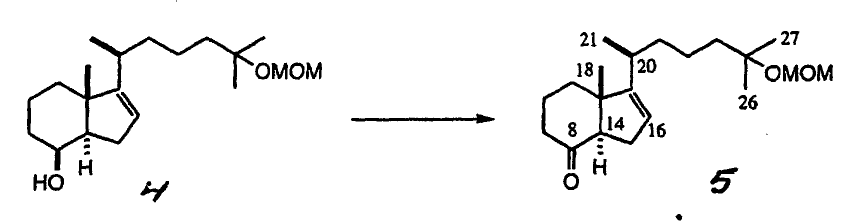

To a solution of 34 mg of **4** (0.105 mmol) in 1.5 ml dry CH₂Cl₂ under an argon atmosphere, was added 109 mg of PDC (0.29 mmol). The mixture was stirred for 6 hrs. The reaction mixture was filtered over Celite and the residue was washed with CH₂Cl₂. The filtrate was concentrated at reduced pressure and the remaining residue was purified by flash chromatography (Eluent 10% EtOAC/hexane) resulting in 32.8 mg of **5** as a colourless oil (97%, Rf = 0.62; 20% EtOAc/hexane).

¹H-NMR (CDCl₃,250 MHz, δ): 5.28 (1 H, t, J=1.2 Hz, H-16),4.68 (2 H, s, OCH₂O), 3.35 (3 H, s, MeO), 2.80 (1 H, dd, J=10.6 Hz and 6.4 Hz, H-14),2.46-2.24 (5 H, m, H-9, H-20 and H-15),1.19 (6 H, s, Me-26 and Me-27),1.05 (3 H, d, J=6.8 Hz, Me-21),0.80 (3 H, s, Me-18).

### (iii) preparation of compound 6 from compound 5.

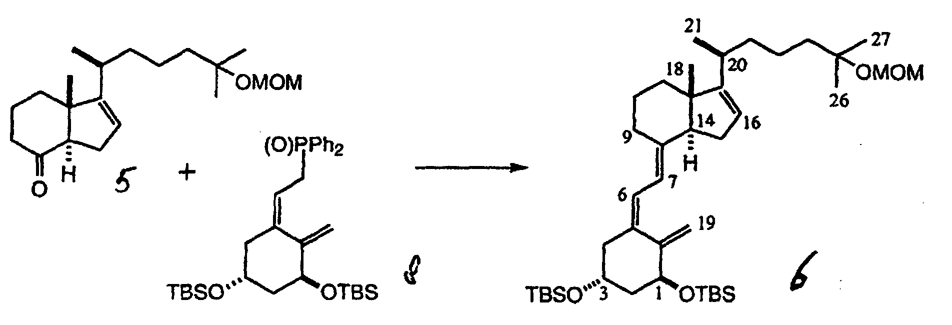

To a cooled (-78 °C) solution of 126 mg of compound 8 (0.216 mmol) in 3 ml of dry THF under an argon atmosphere, was added 0.10 ml of a 2.1 M solution of *n*-BuLi in hexanes (0.216 mmol). After stirring for 1 hr, 35 mg of **5** (0.11 mmol) in 1.6 ml dry THF was added. The reaction mixture was stirred for 12 hrs at -78 °C in the dark and was allowed to reach a temperature of -40 °C in 2 hrs. The reaction was quenched by the addition of 5 ml of water. After separation of the layers, the aqueous layer was extracted with Et₂O (3 x 10 ml). The combined organic layers were washed with 10 ml of a saturated NaCl solution, dried, filtered and concentrated at reduced pressure. The residue was purified by flash chromatography (Eluent 1% EtOAC/hexanes) resulting in 68 mg of **6** as a white solid (92%, Rf = 0.77; 20% EtOAc/hexanes).

### (iv) preparation of compound 7 from compound 6.

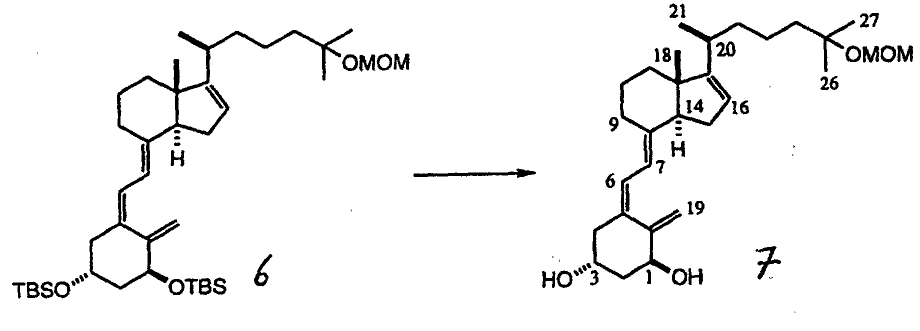

To a solution of 31 mg of **3** (0.045 mmol) in 0.3 ml dry THF under an argon atmosphere, was added 0.84 ml of a 1M solution of TBAF in THF (0.84 mmol). The mixture was stirred in the dark at room temperature for 12 hrs. The reaction was quenched by the addition of 5 ml of a saturated NH₄Cl solution. After separation of the layers, the aqueous layer was extracted with Et₂O (3 x 10 ml). The combined organic layers were dried, filtered and concentrated at reduced pressure. The residue was used directly in the following reaction.

### (v) preparation of compound 9 from compound 7

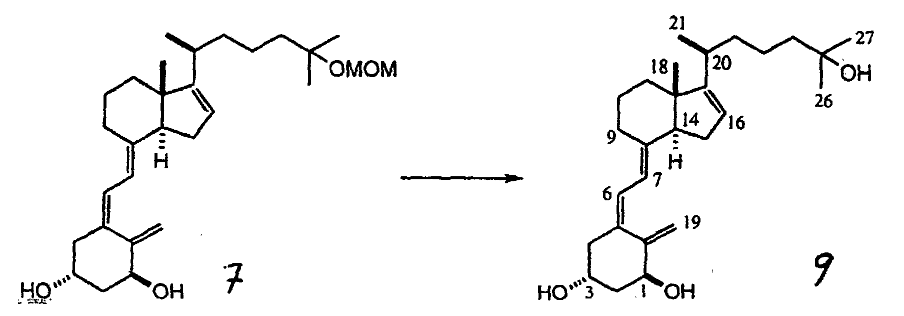

Compound **7** was dissolved in 3 ml of dry MeOH under an argon atmosphere. An amount of 450 mg cationic resin AG50W-X4 (washed with 3 x 10 ml MeOH and dried in vacuo) was added and the mixture was stirred for 5 hrs in the dark. The cationic resin was removed by filtration and washed with dry Et₂O (4 x 10 ml). The filtrate was concentrated at reduced pressure and the remaining residue was purified by flash chromatography (Eluent 50% EtOAC/hexane) resulting in 16 mg of **9** as a white solid (88%, Rf = 0.35; 50% EtOAc/hexane).

¹H-NMR (CDCl₃,250 MHz, δ): 6.35,6.09 (2 H, AB, J=11.1 Hz, H-6 and H-7),5.30 (1 H, s broad, H-16), 5.27 (1 H, s broad, H-19_{E}), 4.90 (1 H, s broad,H-19_{Z}), 4.38 (1 H, m, H-1), 4.15 (1 H, m, H-3), 2.81 (1 H, dd, m, H-14), 1.15 (6 H, s, Me-26 and Me-27), 1.03 (3 H, d, J=6.8 Hz, Me-21),0.69 (3 H, s, Me-18).

According to the process steps i-v the following compounds of formula I wherein Z is (CH₂)₃, R₁=H, A+B=methylene and R₂=OH have been prepared:
a) R= epi-n.butyl
   ¹H-NMR (CDCl₃,250 MHz, δ): 6.35,6.10 (2 H, AB, J=11.1 Hz, H-6 and H-7),5.30 (2 H, s broad, H-16 and H-19_{E}), 4.96 (1 H, s broad,H-19_{Z}),4.37 (1 H, m, H-1), 4.16 (1 H, m, H-3), 2.81 (1 H, m, H-14), 1.15 (6 H, s, Me-26 and Me-27), 0.85 (2 H, m, H-21), 0.67 (3 H, s, Me-18).
b) R=epi-phenyl
   ¹H-NMR (CDCl₃, 250 MHz, δ): 7.26-7.13 (5 H, m, Ph), 6.30, 6.03 (2 H, AB, J=11. 1 Hz, H 6 and H-7), 5.56 (1 H, s broad, H-16),5.26 (1 H, s broad, H-19_{E}), 4.91 (1 H, s broad,H-19_{Z}), 4.35 (1 H, m, H-1), 4.13 (1 H, m, H-3), 3.22 (1 H, t, J=7.5 Hz, H-20), 2.79 (1 H, m, H-14), 1.08 (6 H, s, Me-26 and Me-27), 0.20 (3 H, s, Me-18).
c) R=epi-cyclopropyl
   ¹H-NMR (CDCl₃, 250 MHz, δ): 6.35, 6.10 (2 H, AB, J=11.3 Hz, H-6 and H-7), 5.40 (1 H, s broad, H-16), 5.30 (1 H, s broad, H-19_{E}), 4.96 (1 H, s broad, H-19_{Z}), 4.38 (1 H, m, H-1), 4.16 (1 H, m, H-3),2.78 (1 H, m, H-14),1.15 (6 H, s, Me-26 and Me-27), 0.84 (1 H, m, H-21), 0.69 (3 H, s, Me- 18), 0.56 (1 H, m, CH₂-cylopropyl), 0.40 (1 H, m, CH₂-cyclopropyl), 0.17 (1 H, m, CH₂-cyclopropyl), -0.02 (1 H, m, CH₂-cyclopropyl).
d) R=normal-cyclopropyl
   ¹H-NMR (CDCl₃,250 MHz, δ): 6.35,6.10 (2 H, AB, 1=11.7 Hz, H-6 and H-7),5.34 (1 H, m, H-16),5.29 (1 H, s broad, H-19_{E}), 4.96 (1 H, s broad, H-19_{Z}), 4.38 (1 H, m, H-1), 4.18 (1 H, m, H-3), 2.83 (1 H, m, H-14),1.14 (6 H, s, Me-26 and Me-27), 0.86 (1 H, m, H-21), 0.73 (3 H, s, Me-18), 0.42 (2 H, m, CH₂-cyclopropyl), 0.04. (2 H, m, CH₂-cyclopropyl).
e) R=normal-tert.butyl
   ¹H-NMR (CDCl₃,250 MHz, δ): 6.35,6.12 (2 H, AB, J=11.2 Hz, H-6 and H-7),5.36 (1 H, s broad, H-16),5.29 (1 H, s broad, H-19_{E}), 4.96 (1 H, s broad, H-19_{Z}), 4.36 (1 H, m, H-1), 4.11 (1 H, s broad, H-3), 2.83 (1 H, m, H-14), 1.12 (6 H, s, Me-26 and Me-27), 0.88 (9 H, s, t-Bu), 0.68 (3 H, s, Me-18).
f) R=normal-phenyl
   ¹H-NMR (CDCl₃, 250 MHz, δ): 7.27-7.12 (5 H, m, Ar), 6.35, 6.08 (2 H, AB, J=11.2 Hz, H-6 and H-7), 5.55 (1 H, m, H-16), 5.23 (1 H, s broad, H-19_{E}), 4.97 (1 H, s broad, H-19_{Z}), 4.39 (1 H, m, H-1), 4.18 (1 H, m, H-3), 2.71 (1 H, m, H-14), 1.11 (6 H, s, Me-26 and Me-27), 0.69 (3 H, s, Me- 18).

### Example 3

### Preparation of intermediate 10 of general formula II

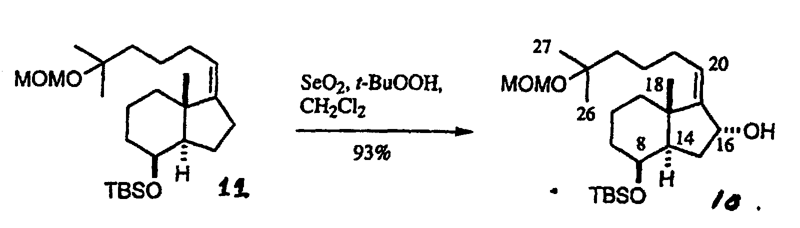

To a cooled (0°C) suspension of 1.1. of freshly sublimed SeO₂ (10.3 mmol) in 30 ml dry CH₂Cl₂ under an argon atmosphere, was added 6.8 ml of a 3 M solution of *t*-BuOOH (20.5 mmol). The mixture was stirred for 1 hr followed by the addition of a solution of 5.7 g of compound 11(13.4 mmol) in 130 ml of dry CH₂Cl₂. After addition was completed, stirring was continued for 6 hrs at room temperature. The reaction was quenched by the addition of 50 ml of a 10% aqueous NaOH solution. The aqueous fase was extracted with CH₂Cl₂(3x25ml). The combined organic layers were dried, filtered and concentrated at reduced pressure. The residue was purified by flash chromatography (eluent 10% EtOAC/hexane) resulting in 5.5 g of compound 10 as a colourless oil (93%, Rf=0.43; 20% EtOAc/hexane).

¹H-NMR (CDCl₃, 250 MHz, δ): 5.38 (1 H, dt, J=7.4 Hz and 1.1 Hz, H-20), 4.69 (2 H, s, OCH₂O), 4.44 (1 H, d, J=6.0 Hz, H-16), 4.09 (1 H, m, H-8), 3.36 (3 H, s, MeO), 2.22-2.12 (3 H, m, H-22 and H-14), 1.20 (6 H, s, Me-26 and Me-27), 1.09 (3 H, s, Me-18), 0.88 (9 H, s, t- BuSi), 0.02 (3 H, s, Me₂Si), 0.01 (3 H, s, Me₂Si).

## Claims

1. A method of preparing a 16-dehydro vitamin D compound of the formula I 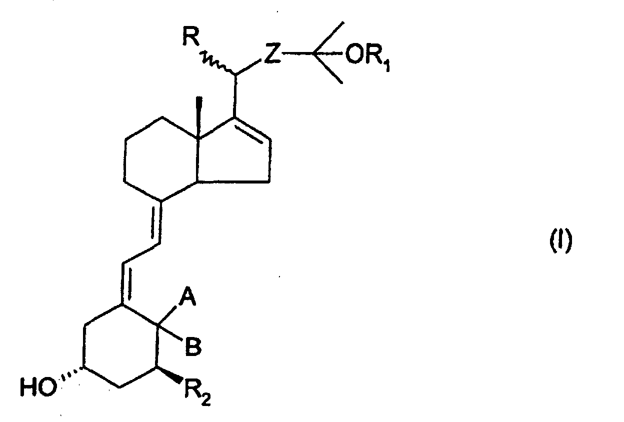 wherein
- R has the epi-configuration or the normal configuration and represents straight or branched C₁₋₆alkyl, C₂₋₆-alkenyl, C₃₋₆-cycloalkyl, or an aromatic group optionally substituted with halogen, C₁₋₃-alkyl or alkoxy,
- Z is a saturated or unsaturated straight hydrocarbon group or oxahydrocarbon group containing 3-6 C-atoms which may be substituted with OH, F, alkyl (C₁₋₃) or cycloalkyl (C₃₋₅),
- R₁ is hydrogen or an hydroxy protecting group,
- R₂ is hydrogen or hydroxy, and
- A and B represent hydrogen or methyl, or together form the methylene group,
**characterised in that** a compound of the formula II 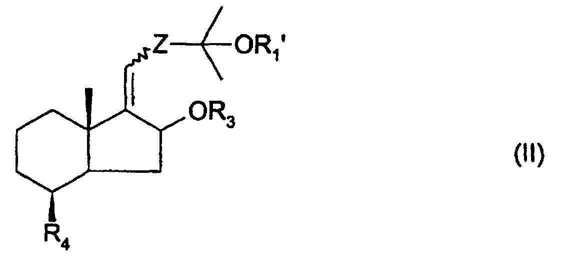 wherein Z has the above meaning, R'₁ is a hydroxy protecting group, R₃ is a carbamate group or pivalate group and R₄ is a protected hydroxy group or a group of the formula III 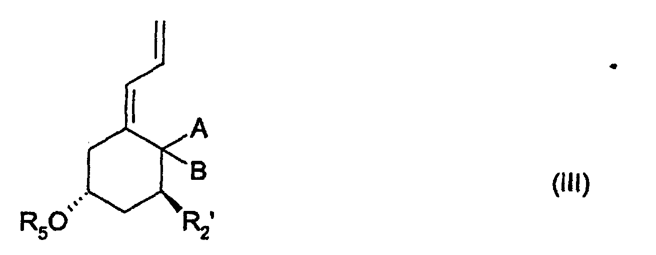 wherein A and B have the above meaning, R₅ is a hydroxy protecting group, and R₂' is hydrogen or a hydroxy protecting group, is converted with a suitable cuprate reagent for introducing group R to a compound of the formula IV 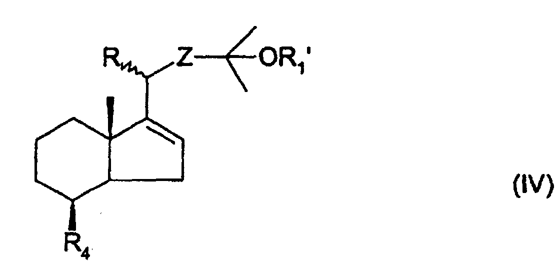 wherein R, R₁', Z and R₄ have the above meaning, followed by converting the obtained compound having formula IV into a compound having formula I in a manner known per se.

2. A 16-dehydro vitamin D compound having formula I wherein R, Z, R₁, A, B and R₂ have the meaning given in claim 1, with the proviso that when R is methyl and Z is a straight hydrocarbon group containing 3 C-atoms, the group R has the epi-configuration.

3. A compound as claimed in claim 2, wherein R is epi-CH₃, epi- or normal cyclopropyl, epi- or normal tert.C₄H₉ or n.C₄H₉, epi- or normal C₆H₅.

4. A pharmaceutical composition comprising as the active ingredient at least one compound according to claim 2.

5. An intermediate having formula IV (a) 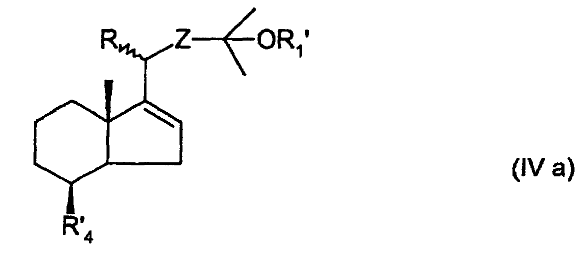 obtainable according to the method of claim 1 wherein R, Z and R₁' have the meaning given in claim 1, and R'₄ is hydroxy, protected hydroxy or keto.

## Patentansprüche

1. Verfahren zum Herstellen einer 16-Dehydro-Vitamin-D-Verbindung der Formel I 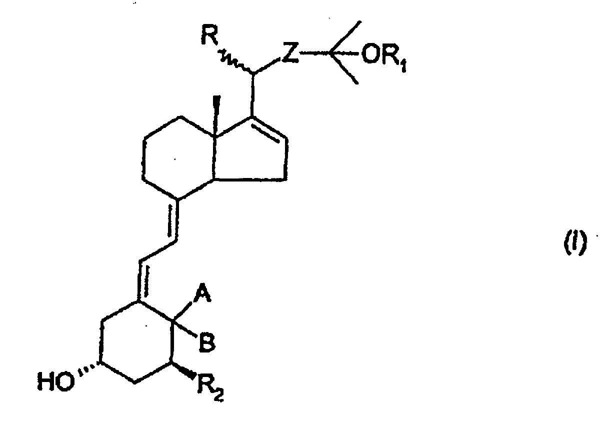 worin
R die epi-Konfiguration oder die normale Konfiguration hat und gerades oder verzweigtes C₁₋₆-Alkyl, C₂₋₆-Alkenyl, C₃₋₆-Cycloalkyl oder eine aromatische Grupe darstellt, gegebenenfalls substituiert mit Halogen, C₁₋₃-Alkyl oder Alkoxy,
Z eine gesättigte oder ungesättigte gerade Kohlenwasserstoffgruppe oder Oxakohlenwasserstoffgruppe darstellt, welche 3-6 C-Atome enthält, welche substituiert sein kann mit OH, F, Alkyl(C₁₋₃) oder Cycloalkyl(C₃₋₅),
R₁ Wasserstoff oder eine Hydroxy-Schutzgruppe darstellt,
R₂ Wasserstoff oder Hydroxy darstellt, und
A und B Wasserstoff oder Methyl darstellen, oder zusammen die Methylengruppe bilden,
**dadurch gekennzeichnet, dass** eine Verbindung der Formel II 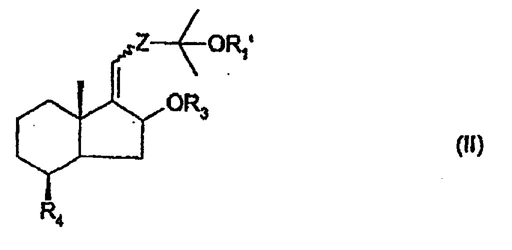 worin Z die obige Bedeutung hat, R₁' eine Hydroxy-Schutzgruppe darstellt, R₃ eine Carbamatgruppe oder Pivalatgruppe darstellt und R₄ eine geschützte Hydroxygruppe darstellt oder eine Gruppe der Formel III 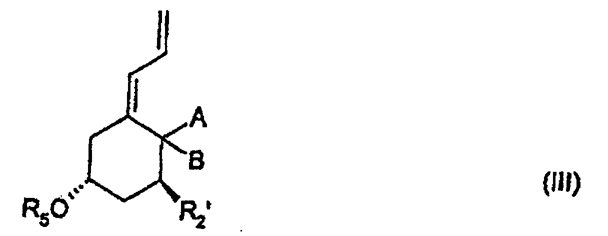 worin A und B die obige Bedeutung haben, R₅ eine Hydroxy-Schutzgruppe darstellt und R₂' Wasserstoff oder eine Hydroxy-Schutzgruppe darstellt,
umgewandelt wird mit einem geeigneten Cuprat-Reagenz zum Einführen von Gruppe R in eine Verbindung der Formel IV 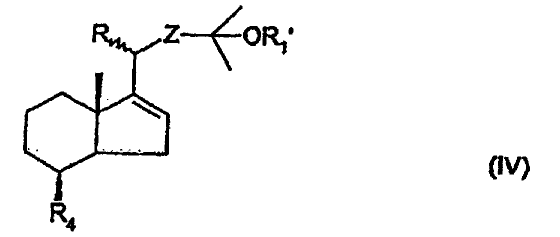 worin R, R₁', Z und R₄ die obige Bedeutung haben, gefolgt von Umwandeln der erhaltenen Verbindung mit der Formel IV in eine Verbindung mit der Formel I auf eine per se bekannte Weise.

2. 16-Dehydro-Vitamin-D-Verbindung mit der Formel I, worin R, Z, R₁, A, B und R₂ die in Anspruch 1 gegebenen Bedeutungen haben, mit der Bedingung, dass wenn R für Methyl steht und Z eine gerade Kohlenwasserstoffgruppe darstellt, welche 3 C-Atome enthält, die Gruppe R die epi-Konfiguration hat.

3. Verbindung wie in Anspruch 2 beansprucht, worin R für epi-CH₃, epi- oder normales Cyclopropyl, epi- oder normales tert.C₄H₉ oder n.C₄H₉, epi- oder normales C₆H₅ steht.

4. Pharmazeutische Zusammenfassung, welche als Wirkstoff mindestens eine Verbindung gemäß Anspruch 2 umfasst.

5. Zwischenverbindung mit der Formel IVa 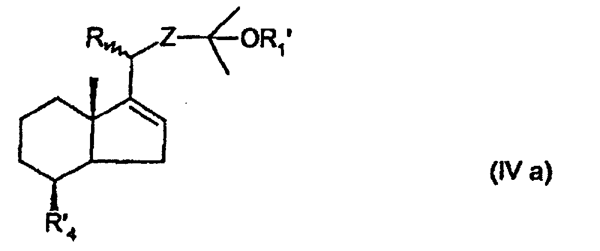 welche gemäß dem Verfahren nach Anspruch 1 erhältlich ist, worin R, Z und R₁' die in Anspruch 1 gegebene Bedeutung haben und R'₄ für Hydroxy, geschütztes Hydroxy oder Keto steht.

## Revendications

1. Procédé de préparation d'un composé de 16-déhydro vitamine D de formule I : 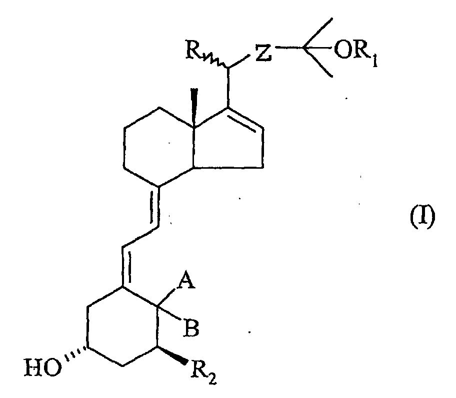 dans laquelle
- R a la configuration épi ou la configuration normale et représente un alkyle en C₁₋₆ linéaire ou ramifié, alcényle en C₂₋₆, cycloalkyle en C₃₋₆, ou un groupe aromatique facultativement substitué par un halogène, un alkyle en C₁₋₃ ou un alcoxy,
- Z est un groupe hydrocarboné ou oxahydrocarboné linéaire, saturé ou insaturé, contenant de 3 à 6 atomes C, pouvant être substitué par OH, F, un alkyle en (C₁₋₃) ou cycloalkyle en (C₃₋₅),
- R₁ est l'hydrogène ou un groupe protecteur hydroxyle,
- R₂ est l'hydrogène ou un groupe hydroxyle, et
- A et B représentent l'hydrogène ou le méthyle, ou forment ensemble le groupe méthylène,
**caractérisé en ce qu'**un composé de formule II 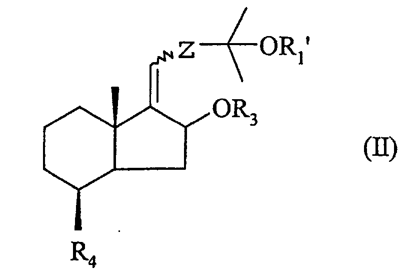 dans laquelle Z a la signification ci-dessus, R'₁ est un groupe protecteur hydroxyle, R₃ est un groupe carbamate ou un groupe pivalate et R₄ est un groupe hydroxyle protégé ou un groupe de formule III 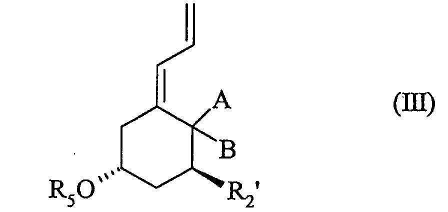 dans laquelle A et B ont la signification ci-dessus, R₅ est un groupe protecteur hydroxyle, et R₂' est l'hydrogène ou un groupe protecteur hydroxyle,
est converti avec un réactif cuprate approprié pour introduire un groupe R dans un composé de formule IV 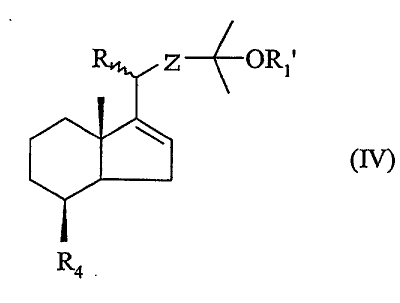 dans laquelle R, R'₁, Z et R₄ ont la signification ci-dessus, après quoi le composé obtenu ayant la formule IV est converti en un composé ayant la formule I d'une manière connue en soi.

2. Composé de 16-déhydro vitamine D ayant la formule I, dans lequel R, Z, R₁, A, B et R₂ ont la signification donnée dans la revendication 1, à la condition que, lorsque R est le méthyle et Z est un groupe hydrocarboné linéaire contenant 3 atomes C, le groupe R a la configuration épi.

3. Composé selon la revendication 2, dans lequel R est l'épi-CH₃, le cyclopropyle épi ou normal, le tert.C₄H₉ ou n.C₄H₉ épi ou normal, le C₆H₅ épi ou normal.

4. Composition pharmaceutique comprenant comme principe actif au moins un composé selon la revendication 2.

5. Intermédiaire ayant la formule IV (a) 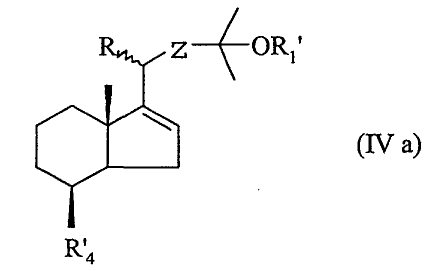 susceptible d'être obtenu selon le procédé de la revendication 1, dans lequel R, Z et R₁' ont la signification donnée dans la revendication 1, et R'₄ est un groupe hydroxyle, hydroxyle protégé ou céto.
